# FASCICULE DE BREVET EUROPEEN

(11) **EP 4 073 812 B1**
(45) Date de publication et mention de la délivrance du brevet: **28.01.2026**
(21) Numéro de dépôt: 20807399.9
(22) Date de dépôt: 18.11.2020
(51) Int. Cl.: G16H 20/17

(54) **SYSTEME DE REGULATION AUTOMATISEE DE GLYCEMIE**
AUTOMATISIERTES SYSTEM ZUR KONTROLLE DES BLUTZUCKERSPIEGELS
AUTOMATED SYSTEM FOR CONTROLLING THE BLOOD GLUCOSE LEVEL

(30) Priorité: 27.11.2019 FR 1913336
(43) Date de publication de la demande: 19.10.2022
(73) Titulaire: Commissariat à l'Energie Atomique et aux Energies Alternatives, 75015 Paris (FR)
(72) Inventeur: VILLENEUVE, Emma, 38054 Grenoble Cedex 9 (FR); DORON, Eléonore-Maeva, 38054 Grenoble Cedex 9 (FR)
(74) Mandataire: Cabinet Beaumont
(86) Numéro de dépôt international: PCT/EP2020/082600
(87) Numéro de publication internationale: WO 2021/104971

(56) Documents cités:
- WO-A1-2017/035019
- US-A1- 2018 085 532

## Description

La présente demande de brevet revendique la priorité de la demande de brevet français FR19/13336 qui sera considérée comme faisant partie intégrante de la présente description.

### Domaine technique

La présente demande concerne le domaine des systèmes automatisés de régulation de glycémie, aussi appelés pancréas artificiels.

### Technique antérieure

On a déjà proposé des systèmes automatisés de régulation de glycémie, aussi appelés pancréas artificiels, permettant de réguler automatiquement les apports en insuline d'un utilisateur ou d'une utilisatrice ("user" en langue anglaise) diabétique à partir de son historique de glycémie (ou taux de glucose sanguin), de son historique de prise de repas, et de son historique d'injection d'insuline.

Des exemples de systèmes de régulation de ce type sont notamment décrits dans les demandes de brevet internationales N°WO2018/055283 (DD16959/B15018), N°WO2018/055284 (DD17175/B15267), N°WO2019/016452 (DD17609/B15860) et N°WO2019/180341 (DD18479/B16770), et dans les demandes de brevet français N°18/56016 du 29 juin 2018 (DD18587/B16893), N°18/00492 du 22 mai 2018 (DD18480/B16894), N°18/00493 du 22 mai 2018 (DD18588/B16895), N°18/73812 du 21 décembre 2018 (DD18986/B17521) et N°19/08457 du 25 juillet 2019 (DD19664/B18647), précédemment déposées par la demanderesse.

Les demandes de brevet WO2017/035019 A1 et US2018/085532 A1 se réfèrent également à des systèmes de régulation de la glycémie d'un patient.

Il serait souhaitable de pouvoir améliorer les performances des pancréas artificiels connus, et notamment de pouvoir limiter davantage les risques de placer l'utilisateur ou l'utilisatrice en situation d'hyperglycémie ou d'hypoglycémie.

On s'intéresse ici plus particulièrement à la gestion des repas non déclarés à l'avance par l'utilisateur ou l'utilisatrice.

### Résumé de l'invention

L'invention est définie par les revendications ci-jointes. Un mode de réalisation prévoit un système automatisé de régulation de glycémie, comportant :
- un capteur de glycémie ;
- un dispositif d'injection d'insuline ; et
- une unité de traitement et de contrôle,
dans lequel l'unité de traitement et de contrôle est configurée pour mettre en oeuvre un procédé de gestion de repas non déclarés, ce procédé comprenant les étapes suivantes :
a) détecter, à un instant t0, un évènement susceptible de correspondre à un repas non déclaré par un utilisateur ou une utilisatrice ;
b) lorsqu'un évènement est détecté à l'étape a), déterminer, à partir d'une première table générée par apprentissage à partir d'un historique de données de l'utilisateur ou l'utilisatrice une probabilité qu'un repas ait été pris par l'utilisateur ou l'utilisatrice dans une période T_ANT de durée prédéterminée précédant l'instant t0 ;
c) si la probabilité déterminée à l'étape b) est supérieure à un seuil TH, déterminer, à partir de la première table, un créneau horaire estimé de prise du repas par l'utilisateur ou l'utilisatrice dans la période T_ANT, et, à partir d'une deuxième table générée par apprentissage à partir d'un historique de données de l'utilisateur ou l'utilisatrice, une taille estimée du repas, puis activer un module de gestion de repas du système de régulation et transmettre audit module le créneau horaire et la taille du repas estimés.

Selon un mode de réalisation :
- la première table est constituée d'une série de valeurs de probabilité correspondant chacune à un pourcentage de fois qu'un repas a été déclaré par l'utilisateur ou par l'utilisatrice dans un intervalle de temps déterminé d'un cycle temporel déterminé, pendant une phase d'apprentissage comprenant une pluralité d'occurrences dudit cycle temporel ; et
- la deuxième table est constituée d'une série de valeurs de taille de repas correspondant chacune à la taille moyenne des repas déclarés par l'utilisateur ou par l'utilisatrice dans chaque intervalle de temps dudit cycle temporel pendant la phase d'apprentissage.

Selon un mode de réalisation, ledit cycle temporel est découpé en une pluralité d'intervalles de temps, le nombre de valeurs de la première table et le nombre de valeurs de la deuxième table étant égaux au nombre d'intervalles de temps du cycle temporel.

Selon un mode de réalisation, l'unité de traitement et de contrôle est configurée pour, si la probabilité déterminée à l'étape b) est inférieure au seuil TH, mettre en oeuvre un procédé de régulation de glycémie non spécifique aux repas.

Selon un mode de réalisation, le système comporte en outre un dispositif d'interface utilisateur relié à l'unité de traitement et de contrôle.

Selon un mode de réalisation, l'unité de traitement et de contrôle est configurée pour, lorsqu'un évènement est détecté à l'étape a), mettre en oeuvre, avant l'étape b), une première étape d'interrogation de l'utilisateur ou l'utilisatrice, au moyen du dispositif d'interface utilisateur, pour lui demander s'il a pris un repas non déclaré dans la période T_ANT.

Selon un mode de réalisation, l'unité de traitement et de contrôle est configurée pour :
- si l'utilisateur ou l'utilisatrice répond négativement à la première interrogation, mettre en oeuvre un procédé de régulation de glycémie non spécifique aux repas ;
- si l'utilisateur ou l'utilisatrice répond positivement à la première interrogation, mettre en oeuvre une deuxième étape d'interrogation de l'utilisateur ou l'utilisatrice, au moyen du dispositif d'interface utilisateur, pour lui demander l'horaire et la taille dudit repas non déclaré pris pendant la période T_ANT ; et
- si l'utilisateur ou l'utilisatrice ne répond pas à la première interrogation, mettre en oeuvre l'étape b) puis l'étape c).

Selon un mode de réalisation, l'unité de traitement est configurée pour :
- si l'utilisateur ou l'utilisatrice répond à la deuxième interrogation, activer le module de gestion de repas du système de régulation et transmettre audit module l'horaire et la taille déclarés par l'utilisateur ou l'utilisatrice en réponse à la première interrogation ; et
- si l'utilisateur ou l'utilisatrice ne répond pas à la deuxième interrogation, déterminer, à partir de la première table, un créneau horaire estimé de prise du repas par l'utilisateur ou l'utilisatrice dans la période T_ANT, et, à partir de la deuxième table, une taille estimée du repas, puis activer le module de gestion de repas du système de régulation et transmettre audit module le créneau horaire et la taille du repas estimés.

Selon un mode de réalisation, l'unité de traitement et de contrôle est configurée pour, à l'étape c), déterminer, au moyen du module de gestion de repas, un bolus d'insuline à injecter à l'utilisateur ou l'utilisatrice en fonction de la taille de repas estimée.

Selon un mode de réalisation, l'unité de traitement et de contrôle est configurée pour, à l'étape c), pondérer le bolus par un facteur d'agressivité fonction de la probabilité déterminée à l'étape b).

Selon un mode de réalisation, les première et deuxième tables sont stockées dans un circuit mémoire de l'unité de traitement et de contrôle.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1 représente de façon schématique, sous forme de blocs, un exemple d'un système automatisé de régulation de la glycémie d'un sujet selon un mode de réalisation ;
la figure 2 est un diagramme illustrant un exemple d'un procédé automatisé de régulation de glycémie pouvant être mis en oeuvre par le système de la figure 1 ;
la figure 3 représente un exemple d'une première table de correspondance pouvant être utilisée pour la mise en oeuvre du procédé de la figure 2 ; et
la figure 4 représente un exemple d'une deuxième table de correspondance pouvant être utilisée pour la mise en oeuvre du procédé de la figure 2.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, les dispositifs de mesure de glycémie et les dispositifs d'injection d'insuline des systèmes de régulation décrits n'ont pas été détaillés, les modes de réalisation décrits étant compatibles avec tous ou la plupart des dispositifs de mesure de glycémie et d'injection d'insuline connus. De plus, la réalisation de l'unité de traitement et de contrôle des systèmes de régulation décrits n'a pas été détaillée, la réalisation d'une telle unité de traitement et de contrôle étant à la portée de la personne du métier à partir des indications fonctionnelles de la présente description.

Sauf précision contraire, les expressions "environ", "approximativement", "sensiblement", et "de l'ordre de" signifient à 10 % près, de préférence à 5 % près.

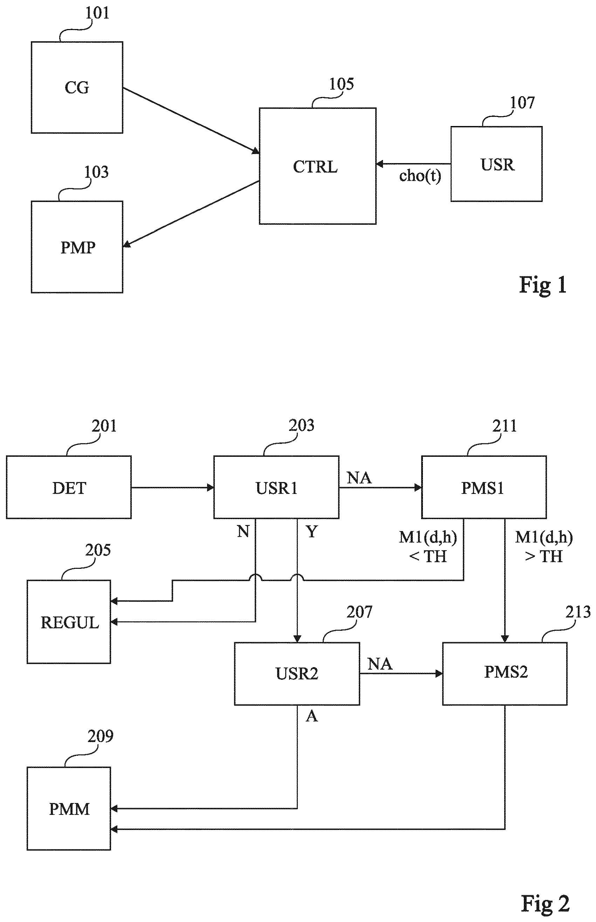

La figure 1 représente de façon schématique, sous forme de blocs, un exemple d'un mode de réalisation d'un système automatisé de régulation de la glycémie d'un utilisateur ou d'une utilisatrice.

Le système de la figure 1 comprend un capteur 101 (CG) adapté à mesurer une grandeur représentative de la glycémie de l'utilisateur ou l'utilisatrice, par exemple la concentration de glucose dans le liquide interstitiel, que l'on appellera ci-après glycémie par souci de simplification. En fonctionnement normal, le capteur 101 peut être positionné à demeure sur ou dans le corps de l'utilisateur ou l'utilisatrice, par exemple à hauteur de son abdomen ou au niveau de son bras. Le capteur 101 est par exemple un capteur de type CGM (de l'anglais "Continuous Glucose Monitoring" - surveillance continue de glycémie), c'est-à-dire un capteur adapté à mesurer en continu ou à une fréquence relativement élevée (par exemple au moins une fois toutes les vingt minutes et de préférence au moins une fois toutes les cinq minutes) la glycémie de l'utilisateur ou l'utilisatrice. Le capteur 101 est par exemple un capteur de glycémie en sous-cutané.

Le système de la figure 1 comprend en outre un dispositif d'injection d'insuline 103 (PMP), par exemple un dispositif d'injection en sous-cutané. Le dispositif 103 est par exemple un dispositif d'injection automatique de type pompe à insuline, comportant un réservoir d'insuline relié à une aiguille d'injection implantée sous la peau de l'utilisateur ou l'utilisatrice, la pompe pouvant être commandée électriquement pour injecter automatiquement des doses d'insuline déterminées à des instants déterminés. En fonctionnement normal, le dispositif d'injection 103 peut être positionné à demeure dans ou sur le corps de l'utilisateur ou l'utilisatrice, par exemple au niveau de son abdomen.

Le système de la figure 1 comprend en outre une unité de traitement et de contrôle 105 (CTRL) reliée d'une part au capteur de glycémie 101, par exemple par liaison filaire ou par liaison radio (sans fil), et d'autre part au dispositif d'injection 103, par exemple par liaison filaire ou radio. En fonctionnement, l'unité de traitement et de contrôle 105 est adaptée à recevoir les données de glycémie de l'utilisateur ou l'utilisatrice mesurées par le capteur 101, et à commander électriquement le dispositif 103 pour injecter à l'utilisateur ou l'utilisatrice des doses d'insuline déterminées à des instants déterminés. Dans cet exemple, l'unité de traitement et de contrôle 105 est en outre adaptée à recevoir, par l'intermédiaire d'une interface utilisateur 107 (USR), des données cho(t) représentatives de l'évolution, en fonction du temps, de la quantité de glucose ingérée par l'utilisateur ou l'utilisatrice.

L'unité de traitement et de contrôle 105 est adaptée à déterminer les doses d'insuline à injecter à l'utilisateur ou l'utilisatrice en tenant compte notamment de l'historique de glycémie mesurée par le capteur 101, de l'historique d'insuline injectée par le dispositif 103, et de l'historique d'ingestion de glucose par l'utilisateur ou l'utilisatrice. Pour cela, l'unité de traitement et de contrôle 105 comprend un circuit de calcul numérique (non détaillé), comprenant par exemple un microprocesseur. L'unité de traitement et de contrôle 105 est par exemple un dispositif mobile transporté par l'utilisateur ou l'utilisatrice tout au long de la journée et/ou de la nuit, par exemple un dispositif de type smartphone configuré pour mettre en oeuvre un procédé de régulation du type décrit ci-après.

L'unité de traitement et de contrôle 105 est par exemple configurée pour, en dehors des périodes de repas, mettre en oeuvre un procédé automatisé de régulation de type MPC (de l'anglais "Model-based Predictive Control" - commande prédictive basée sur un modèle), aussi appelé procédé de régulation à commande prédictive, dans lequel la régulation de la dose d'insuline administrée tient compte d'une prédiction de l'évolution future de la glycémie de l'utilisateur ou l'utilisatrice en fonction du temps, réalisée à partir d'un modèle mathématique, par exemple un modèle physiologique décrivant l'assimilation de l'insuline par le corps de l'utilisateur ou l'utilisatrice et son impact sur sa glycémie. Plus particulièrement, l'unité de traitement et de contrôle 105 peut être configurée pour, à partir de l'historique d'insuline injectée et de l'historique de glucose ingéré, et en se basant sur un modèle mathématique prédéterminé, déterminer une courbe représentative de l'évolution attendue de la glycémie de l'utilisateur ou l'utilisatrice en fonction du temps, sur une période à venir appelée période de prédiction ou horizon de prédiction, par exemple une période de 1 à 10 heures. En tenant compte de cette courbe, l'unité de traitement et de contrôle 105 détermine les doses d'insuline qu'il conviendrait d'injecter à l'utilisateur ou l'utilisatrice pendant la période de prédiction à venir, pour que la glycémie réelle (par opposition à la glycémie estimée à partir du modèle) de l'utilisateur ou l'utilisatrice reste dans des limites acceptables, et en particulier pour limiter les risques d'hyperglycémie ou d'hypoglycémie.

A titre de variante, l'unité de traitement et de contrôle 105 peut être configurée pour, en dehors des périodes de repas, mettre en oeuvre un procédé automatisé de régulation de glycémie de type matrice décisionnelle, pour déterminer les doses d'insuline à administrer à l'utilisateur ou l'utilisatrice en fonction de divers paramètres observés tels que le niveau courant de glycémie mesuré par le capteur 101, ou encore la vitesse de variation (ou pente) de la glycémie sur une période passée.

Dans une autre variante, l'unité de traitement et de contrôle 105 peut être configurée pour, en dehors des périodes de repas, alterner entre un procédé de régulation de type MPC et un procédé de régulation de type matrice décisionnelle.

En principe, l'utilisateur ou l'utilisatrice déclare chacun de ses repas, et en particulier l'heure de prise du repas et la quantité de glucose approximative ingérée lors du repas (aussi appelée taille du repas), par le biais de l'interface utilisateur 107.

L'unité de traitement et de contrôle 105 est configurée pour, lorsqu'un repas est déclaré par l'utilisateur ou l'utilisatrice, activer un module spécifique de gestion de repas, ce module mettant en oeuvre un procédé de régulation adapté à tenir compte des spécificités physiologiques liées à l'assimilation d'un repas. Le module de gestion de repas est par exemple implémenté sous forme logicielle au moyen de l'unité de traitement et de contrôle 105. Le procédé de régulation mis en oeuvre par le module de gestion de repas peut comprendre une étape de calcul, à partir des données saisies par l'utilisateur ou l'utilisatrice, et notamment de l'heure déclarée et de la taille déclarée du repas, d'un bolus d'insuline, c'est-à-dire d'une dose d'insuline complémentaire à injecter à l'utilisateur ou l'utilisatrice en supplément d'un débit de base d'insuline normalement injecté. Le module de gestion de repas commande ensuite l'injection du bolus par le dispositif d'injection 103. Le bolus peut être administré en une seule injection ou en plusieurs injections successives, par exemple en deux injections. Le bolus peut être administré au moment de la déclaration du repas, ou au début du repas voire un peu avant le début du repas si la déclaration intervient avant le début du repas. Si le repas est annoncé avec retard, le bolus peut être administré après le début du repas. A titre d'exemple, le bolus d'insuline injecté lors d'un repas peut être au moins deux fois supérieur à la dose d'insuline normalement injectée en une heure en dehors des périodes de repas. A titre d'exemple, le débit de base d'insuline normalement injecté à l'utilisateur ou l'utilisatrice en dehors des périodes de repas est compris entre 0,3 et 1,5 UI/h, où UI désigne une unité internationale d'insuline, soit l'équivalent biologique d'environ 0,0347 mg d'insuline humaine. Le bolus déterminé par le module de gestion de repas puis injecté par le dispositif d'injection 103 est par exemple compris entre 3 et 30 UI en fonction de la taille déclarée du repas et de la sensibilité à l'insuline du sujet. Après injection du bolus, le module de gestion de repas peut moduler le débit de base d'insuline injecté à l'utilisateur ou l'utilisatrice, par exemple au moyen d'un filtre PID ou correcteur PID ("Proportionnel Intégral Dérivé"), pendant une période de durée prédéterminée, par exemple une période de trois heures suivant l'injection du bolus, de manière à ramener la glycémie courante à une valeur cible. A l'issue de cette période de modulation, le procédé de gestion de repas se termine. L'unité de traitement et de contrôle 105 peut alors mettre en oeuvre un autre procédé de régulation de glycémie, par exemple un procédé de type MPC ou de type matrice décisionnelle tel que décrit ci-dessus.

Une limitation du fonctionnement décrit ci-dessus est que son efficacité dépend fortement des déclarations de l'utilisateur ou l'utilisatrice. Si l'utilisateur ou l'utilisatrice omet de déclarer un repas, le module de gestion de repas n'est pas activé. La glycémie de l'utilisateur ou l'utilisatrice est alors assurée par un procédé de régulation non spécifique aux repas, par exemple un procédé de type MPC ou de type matrice décisionnelle tel que décrit ci-dessus, ce qui peut conduire à des périodes d'hyperglycémie relativement longues, notamment en raison du manque d'agressivité de ces procédés face à des situations d'hyperglycémie liées à une prise de repas.

La figure 2 est un diagramme illustrant un exemple d'un procédé de régulation de glycémie adapté à gérer des repas non déclarés par l'utilisateur ou l'utilisatrice. Ce procédé est basé sur l'utilisation de données statistiques générées par apprentissage à partir d'un historique de données concernant l'utilisateur ou l'utilisatrice.

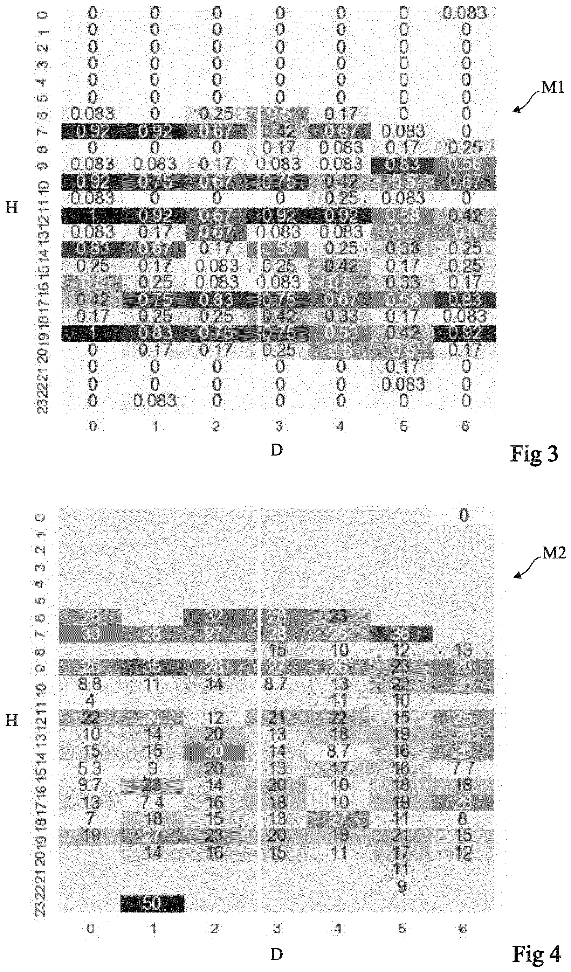

Le procédé de la figure 2 est basé plus particulièrement sur l'utilisation d'une table ou matrice de probabilité de repas M1, du type illustré par la figure 3, et d'une table ou matrice de taille moyenne de repas M2, du type illustré par la figure 4.

Dans l'exemple de la figure 3, la table M1 comprend un nombre entier H de rangées et un nombre entier D de colonnes. Dans l'exemple représenté, le nombre D est égal à 7, chaque colonne de la table M1 correspondant à un jour de la semaine. De plus, dans cet exemple, le nombre H est égal à 24, chaque rangée de la table M1 correspondant à une heure de la journée.

Dans l'exemple de la figure 4, la table M2 comprend le même nombre H de rangées et le même nombre D de colonnes que la table M1. Chaque colonne de la table M2 correspond à un jour de la semaine, et chaque rangée de la table M2 correspond à une heure de la journée.

En désignant par d le rang des colonnes des tables M1 et M2, avec d entier allant de 0 à 6, et par h le rang des rangées des tables M1 et M2, avec h entier allant de 0 à 23, chaque valeur M1(d, h) de coordonnées (d, h) dans la table M1 correspond à la probabilité qu'un repas ait été pris par l'utilisateur ou l'utilisatrice le jour d dans le créneau horaire h, et chaque valeur M2 (d, h) de cordonnées (d, h) dans la table M2 correspond à la taille moyenne des repas habituellement pris par l'utilisateur ou l'utilisatrice le jour d dans le créneau horaire h, par exemple en gCHO, c'est-à-dire en grammes de glucides. Dans cet exemple, les jours de rang d=0 à d=6 correspondent respectivement aux sept jours de la semaine, et chaque créneau horaire de rang h correspond à un créneau d'une heure allant de l'heure h à l'heure h+1.

Les tables M1 et M2 peuvent être générées par apprentissage à partir d'un historique de données acquis pour l'utilisateur ou l'utilisatrice pendant une phase préalable d'apprentissage, par exemple une phase de plusieurs jours à plusieurs semaines. A titre d'exemple, chaque valeur M1(d, h) de la table M1 correspond au pourcentage de fois qu'un repas a été déclaré par l'utilisateur ou l'utilisatrice le jour d et dans le créneau h pendant la phase d'apprentissage. Chaque valeur M2 (d, h) de la table M2 correspond par exemple à la taille moyenne des repas déclarés par l'utilisateur ou l'utilisatrice le jour d et dans le créneau h pendant la phase d'apprentissage.

Les tables M1 et M2 peuvent être stockées dans un circuit mémoire du dispositif de traitement et de contrôle 105.

Les tables M1 et M2 peuvent par exemple être mises à jour au fur et à mesure de l'utilisation du système, à chaque fois que l'utilisateur ou l'utilisatrice déclare un repas via l'interface utilisateur 107.

Lorsque des tables M1 et M2 personnalisées ne sont pas disponibles pour un sujet donné, on pourra, en première approche, utiliser des tables M1 et M2 génériques, obtenues à partir d'un historique de données de population. Des tables génériques M1 et M2 peuvent par exemple être déterminées pour plusieurs types de population, par exemple des enfants scolarisés, des adolescents, des adultes, éventuellement avec différents rythmes de repas, par exemple en fonction du pays de résidence.

Le procédé de la figure 2 comprend une étape 201 (DET) de détection d'un évènement susceptible de correspondre à un repas non déclaré par l'utilisateur ou l'utilisatrice. A titre d'exemple, la détection mise en oeuvre à l'étape 201 peut être basée sur des mesures fournies par le capteur de glycémie 101 du système. Un évènement détecté à l'étape 201 correspond par exemple à une montée de la glycémie de l'utilisateur ou l'utilisatrice du type normalement observée suite à une ingestion d'aliments, mais non expliquée par une déclaration antérieure d'un repas par l'utilisateur ou l'utilisatrice. A titre d'exemple, l'unité de traitement et de contrôle 105 peut être configurée pour mettre en oeuvre une surveillance continue de la courbe de glycémie de l'utilisateur ou l'utilisatrice en vue de détecter de tels évènements.

Lorsque, à un instant t0, un évènement est détecté à l'étape 201, une première étape 203 (USR1) d'interrogation de l'utilisateur ou l'utilisatrice au moyen du dispositif d'interface utilisateur 107 est mise en oeuvre. Lors de cette étape, il est demandé à l'utilisateur ou l'utilisatrice, par l'intermédiaire du dispositif 107, s'il a pris un repas dans une période T_ANT de durée prédéterminée précédant l'instant t0, par exemple dans les trois heures précédant l'instant t0.

Si, à l'étape 203, l'utilisateur ou l'utilisatrice répond, par l'intermédiaire du dispositif 107, qu'il n'a pas (N) pris de repas non déclaré dans la période T_ANT considérée, la régulation se poursuit lors d'une étape 205 (REGUL) avec un procédé de régulation non spécifique aux repas, par exemple un procédé de type MPC ou de type matrice décisionnelle tel que décrit ci-dessus.

Si, à l'étape 203, l'utilisateur ou l'utilisatrice répond qu'il a (Y) pris un repas non déclaré dans la période T_ANT considérée, une deuxième étape 207 (USR2) d'interrogation de l'utilisateur ou l'utilisatrice au moyen du dispositif d'interface utilisateur 107 est mise en oeuvre. Lors de cette étape, il est demandé à l'utilisateur ou l'utilisatrice, par l'intermédiaire du dispositif 107, la taille du repas non déclaré et l'heure de prise du repas non déclaré. Autrement dit, lors des étapes 203 et 207, il est demandé à l'utilisateur ou l'utilisatrice de déclarer a postériori le repas qu'il avait omis de déclarer.

Si, à l'étape 207, l'utilisateur ou l'utilisatrice répond (A), par l'intermédiaire du dispositif 107, en indiquant la taille et l'heure du repas non déclaré, l'unité de traitement et de contrôle 105 active un module spécifique de gestion de repas, ce module mettant en oeuvre, lors d'une étape 209 (PMM), un procédé de régulation adapté à tenir compte des spécificités physiologiques liées à l'assimilation d'un repas, en tenant compte de la taille de repas et de l'heure de repas déclarées par l'utilisateur ou l'utilisatrice.

Si, à l'étape 203, l'utilisateur ou l'utilisatrice ne répond pas (NA) à la question posée concernant la prise éventuelle d'un repas non déclaré dans la période T_ANT, une étape 211 (PMS1) est mise en oeuvre, au cours de laquelle l'unité de traitement et de contrôle 105 détermine, à partir de la table M1, si la probabilité qu'un repas non déclaré ait été pris par l'utilisateur ou l'utilisatrice dans la période T_ANT est supérieure ou non à un seuil TH prédéterminé. Pour cela, l'unité de traitement et de contrôle 105 détermine si la matrice M1 contient une valeur de probabilité M1(d, h) supérieure au seuil TH dans la colonne de rang d correspondant au jour courant et dans les rangées correspondant à la plus petite plage horaire comprenant la période T_ANT.

Si, à l'étape 211, il est déterminé que la table M1 ne comprend pas de valeur de probabilité de prise de repas M1(d, h) supérieure au seuil TH dans la plage T_ANT, l'étape 205 est mise en oeuvre, c'est-à-dire que la régulation se poursuit avec un procédé de régulation non spécifique aux repas, par exemple un procédé de type MPC ou de type matrice décisionnelle tel que décrit ci-dessus.

Si, à l'étape 211, il est déterminé que la table M1 comprend une valeur de probabilité de prise de repas M1(d, h) supérieure au seuil TH dans la plage T_ANT, une étape 213 (PMS2) est mise en oeuvre, au cours de laquelle l'unité de traitement et de contrôle 105 détermine un créneau horaire estimé de prise de repas par l'utilisateur ou l'utilisatrice dans la période T_ANT, et une taille estimée du repas pris.

Pour déterminer le créneau horaire estimé de prise de repas par l'utilisateur ou l'utilisatrice, l'unité de traitement et de contrôle 105 peut utiliser la table M1. Le créneau horaire estimé correspond par exemple au créneau de coordonnées d, h de la période T_ANT pour lequel la valeur de probabilité de prise de repas M1(d, h) de la table M1 est la plus élevée. A titre de variante, le repas peut être positionné à l'heure où l'évènement a été identifié à l'étape 201.

Pour déterminer la taille estimée du repas pris, l'unité de traitement et de contrôle 105 peut utiliser la table M2. La taille estimée du repas correspond par exemple à la valeur M2 (d, h) de la table M2 dans le créneau horaire de coordonnées d, h estimé à partir de la table M1. A titre de variante, la taille estimée du repas peut être déterminée à partir de la montée glycémique observée.

A l'issue de l'étape 213, l'étape 209 est mise en oeuvre, c'est-à-dire que l'unité de traitement et de contrôle 105 active un module spécifique de gestion de repas, ce module mettant en oeuvre un procédé de régulation adapté à tenir compte des spécificités physiologiques liées à l'assimilation d'un repas. Dans ce cas, le procédé de régulation mis en oeuvre à l'étape 209 prend comme paramètres d'entrée la taille estimée et le créneau horaire de repas estimé déterminés à l'étape 213 à partir des tables M1 et M2.

Si, à l'étape 207, l'utilisateur ou l'utilisatrice ne répond pas (NA) à la question posée quant à l'heure et à la taille du repas non déclaré pris pendant la période T_ANT, l'étape 213 est mise en oeuvre de façon à estimer le créneau horaire et la taille du repas non déclaré à partir des tables M1 et M2. L'étape 209 est ensuite mise en oeuvre de façon similaire à ce qui vient d'être décrit.

De préférence, le bolus d'insuline à injecter à l'utilisateur ou l'utilisatrice, déterminé par le procédé de régulation mis en oeuvre à l'étape 209, est pondéré par un facteur d'agressivité, ce facteur pouvant prendre une première valeur lorsque l'étape 209 est mise en oeuvre suite à une déclaration de repas effectuée par l'utilisateur ou l'utilisatrice à l'étape 207, et une deuxième valeur inférieure à la première valeur lorsque l'étape 209 est mise en oeuvre suite à une estimation de taille et d'heure de repas à partir des tables M1 et M2 à l'étape 213. Dans le cas où l'étape 211 est mise en oeuvre, la deuxième valeur peut être d'autant plus faible que la probabilité qu'un repas ait été pris par l'utilisateur ou l'utilisatrice dans la période T_ANT est faible.

Le procédé décrit en relation avec la figure 2 permet avantageusement, en utilisant des données statistiques représentatives des heures et tailles des repas habituellement pris par l'utilisateur ou l'utilisatrice, de traiter en tant que repas, au moyen d'un module de régulation spécifique, des évènements susceptibles de correspondre à des repas mais non déclarés en tant que tels par l'utilisateur ou l'utilisatrice.

A titre de variante, les étapes 203 et 207 d'interrogation de l'utilisateur ou l'utilisatrice peuvent être omises. Dans ce cas, lorsqu'un évènement susceptible de correspondre à un repas est détecté à l'étape 201, l'étape 211 est directement mise en oeuvre, suivie de l'étape 205 s'il est déterminé à l'étape 211 que la probabilité qu'un repas non déclaré ait été pris par l'utilisateur ou l'utilisatrice pendant la période antérieure T_ANT précédant l'instant t0 de détection de l'évènement est inférieure au seuil TH, ou suivie de l'étape 213 puis de l'étape 209 dans le cas contraire.

Divers modes de réalisation et variantes ont été décrits. La personne du métier comprendra que certaines caractéristiques de ces divers modes de réalisation et variantes pourraient être combinées, et d'autres variantes apparaîtront à la personne du métier. En particulier, les modes de réalisation décrits ne se limitent pas aux exemples décrits en relation avec les figures 3 et 4 de granularité de découpage temporel des axes horizontaux et verticaux des tables M1 et M2. A titre de variante, plutôt que d'avoir une valeur de probabilité de repas et une valeur de taille moyenne de repas par heure et par jour sur sept jours consécutifs dans les tables M1 et M2, on pourra prévoir des tables M1 et M2 comportant une valeur de probabilité de repas et une valeur de taille moyenne de repas par heure et par jour de travail (indépendamment du jour considéré) et une valeur de probabilité de repas et une valeur de taille moyenne de repas par heure et par jour non travaillé (indépendamment du jour considéré). Plus généralement, tout autre découpage adapté au mode de vie de l'utilisateur ou l'utilisatrice pourra être considéré.

Par ailleurs, les modes de réalisation décrits ne se limitent pas à l'exemple décrit en relation avec la figure 2 dans lequel la détection mise en oeuvre à l'étape 201 est basée sur des mesures de glycémie fournies par le capteur 101. Plus généralement, la détection d'évènements susceptibles de correspondre à des repas mise en oeuvre à l'étape 201 peut être basée sur tout autre indicateur approprié, en complément ou en remplacement de mesures de glycémie.

## Revendications

1. Système automatisé de régulation de glycémie, comportant :
- un capteur de glycémie (101) ;
- un dispositif d'injection d'insuline (103) ; et
- une unité de traitement et de contrôle (105),
dans lequel l'unité de traitement et de contrôle est configurée pour mettre en oeuvre un procédé de gestion de repas non déclarés, ce procédé comprenant les étapes suivantes :
a) détecter (201), à un instant t0, sur la base de mesures fournies par le capteur de glycémie (101) ou d'un autre indicateur, un évènement susceptible de correspondre à un repas non déclaré par un utilisateur ou une utilisatrice ;
b) lorsqu'un évènement est détecté à l'étape a), lire (211), dans une première table (M1) générée par apprentissage à partir d'un historique de données de l'utilisateur ou l'utilisatrice, une probabilité qu'un repas ait été pris par l'utilisateur ou l'utilisatrice dans une période T_ANT de durée prédéterminée précédant l'instant t0 ;
c) si la probabilité lue à l'étape b) est supérieure à un seuil TH, lire (213), dans la première table (M1), un créneau horaire estimé de prise du repas par l'utilisateur ou l'utilisatrice dans la période T_ANT, et, lire, dans une deuxième table (M2) générée par apprentissage à partir d'un historique de données de l'utilisateur ou l'utilisatrice, une taille estimée du repas, puis activer (209) un module de gestion de repas du système de régulation et transmettre audit module le créneau horaire et la taille du repas estimés,
dans lequel :
- la première table (M1) est constituée d'une série de valeurs de probabilité correspondant chacune à un pourcentage de fois qu'un repas a été déclaré par l'utilisateur ou par l'utilisatrice dans un intervalle de temps déterminé d'un cycle temporel déterminé, pendant une phase d'apprentissage comprenant une pluralité d'occurrences dudit cycle temporel ; et
- la deuxième table (M2) est constituée d'une série de valeurs de taille de repas correspondant chacune à la taille moyenne des repas déclarés par l'utilisateur ou par l'utilisatrice dans chaque intervalle de temps dudit cycle temporel pendant la phase d'apprentissage.

2. Système selon la revendication 1, dans lequel le premier cycle temporel est découpé en une pluralité d'intervalles de temps, le nombre de valeurs de la première table (M1) et le nombre de valeurs de la deuxième table (M2) étant égaux au nombre d'intervalles de temps dudit cycle temporel.

3. Système selon la revendication 1 ou 2, dans lequel l'unité de traitement et de contrôle (105) est configurée pour, si la probabilité déterminée à l'étape b) est inférieure au seuil TH, mettre en oeuvre (205) un procédé de régulation de glycémie non spécifique aux repas.

4. Système selon l'une quelconque des revendications 1 à 3, comportant en outre un dispositif d'interface utilisateur (107) relié à l'unité de traitement et de contrôle (105).

5. Système selon la revendication 4, dans lequel l'unité de traitement et de contrôle (105) est configurée pour, lorsqu'un évènement est détecté à l'étape a), mettre en oeuvre, avant l'étape b), une première étape (203) d'interrogation de l'utilisateur ou l'utilisatrice, au moyen du dispositif d'interface utilisateur (107), pour lui demander s'il a pris un repas non déclaré dans la période T_ANT.

6. Système selon la revendication 5, dans lequel l'unité de traitement et de contrôle (105) est configurée pour :
- si l'utilisateur ou l'utilisatrice répond négativement à la première interrogation, mettre en oeuvre (205) un procédé de régulation de glycémie non spécifique aux repas ;
- si l'utilisateur ou l'utilisatrice répond positivement à la première interrogation, mettre en oeuvre une deuxième étape (207) d'interrogation de l'utilisateur ou l'utilisatrice, au moyen du dispositif d'interface utilisateur (107), pour lui demander l'horaire et la taille dudit repas non déclaré pris pendant la période T_ANT ; et
- si l'utilisateur ou l'utilisatrice ne répond pas à la première interrogation, mettre en oeuvre l'étape b) puis l'étape c).

7. Système selon la revendication 6, dans lequel l'unité de traitement (105) est configurée pour :
- si l'utilisateur ou l'utilisatrice répond à la deuxième interrogation, activer (209) le module de gestion de repas du système de régulation et transmettre audit module l'horaire et la taille déclarés par l'utilisateur ou l'utilisatrice en réponse à la deuxième interrogation ; et
- si l'utilisateur ou l'utilisatrice ne répond pas à la deuxième interrogation, déterminer (213), à partir de la première table (M1), un créneau horaire estimé de prise du repas par l'utilisateur ou l'utilisatrice dans la période T_ANT, et, à partir de la deuxième table (M2), une taille estimée du repas, puis activer (209) le module de gestion de repas du système de régulation et transmettre audit module le créneau horaire et la taille du repas estimés.

8. Système selon l'une quelconque des revendications 1 à 7, dans lequel l'unité de traitement et de contrôle (105) est configurée pour, à l'étape c), déterminer, au moyen du module de gestion de repas, un bolus d'insuline à injecter à l'utilisateur ou l'utilisatrice en fonction de la taille de repas estimée.

9. Système selon la revendication 8, dans lequel l'unité de traitement et de contrôle (105) est configurée pour, à l'étape c), pondérer ledit bolus par un coefficient fonction de la probabilité déterminée à l'étape b).

10. Système selon l'une quelconque des revendications 1 à 9, dans lequel les première (M1) et deuxième (M2) tables sont stockées dans un circuit mémoire de l'unité de traitement et de contrôle (105).

## Patentansprüche

1. Automatisiertes System zur Blutglukoseregulation, das Folgendes aufweist:
- einen Blutglukosesensor (101);
- eine Insulininjektionsvorrichtung (103); und
- eine Verarbeitungs- und Steuereinheit (105),
wobei die Verarbeitungs- und Steuereinheit so konfiguriert ist, dass sie ein Verfahren zur Verwaltung nicht gemeldeter Mahlzeiten implementiert, wobei dieses Verfahren die folgenden Schritte aufweist:
a) Erkennen (201), und zwar zu einem Zeitpunkt t0, auf Grundlage von Messungen, die vom Blutglukosesensor (101) oder einem anderen Indikator bereitgestellt werden, eines Ereignisses, das wahrscheinlich einer von einem Benutzer nicht gemeldeten Mahlzeit entspricht;
b) wenn in Schritt a) ein Ereignis erkannt wird, Auslesen (211), und zwar in einer ersten Tabelle (M1), die durch Training anhand einer Historie von Daten des Benutzers erstellt wurde, einer Wahrscheinlichkeit dafür, dass der Benutzer innerhalb eines Zeitraums T_ANT mit vorbestimmter Dauer vor einem Zeitpunkt t0 eine Mahlzeit eingenommen hat;
c) falls die in Schritt b) ausgelesene Wahrscheinlichkeit größer als ein Schwellenwert TH ist, Auslesen (213), und zwar in der ersten Tabelle (M1), eines geschätzten Zeitabschnittes für eine Mahlzeitaufnahme durch den Benutzer innerhalb des Zeitraums T_ANT, und Auslesen, und zwar in einer zweiten Tabelle (M2), die durch Training anhand einer Historie von Daten des Benutzers erstellt wurde, einer geschätzten Größe der Mahlzeit, und dann Aktivieren (209) eines Moduls zum Mahlzeitenmanagement des Systems zur Regulation und Übertragen des geschätzten Zeitabschnittes und der geschätzten Größe der Mahlzeit an das Modul,
wobei:
- die erste Tabelle (M1) aus einer Reihe von Wahrscheinlichkeitswerten gebildet ist, die jeweils einem Prozentsatz der Fälle entsprechen, in denen eine Mahlzeit vom Benutzer innerhalb eines bestimmten Zeitintervalls eines bestimmten Zeitzyklus während einer Trainingsphase, die eine Vielzahl von Auftritten dieses Zeitzyklus aufweist, angegeben wurde; und
- die zweite Tabelle (M2) aus einer Reihe von Mahlzeitgrößenwerten gebildet ist, die jeweils der durchschnittlichen Mahlzeitgröße entsprechen, die vom Benutzer innerhalb jedes Zeitintervalls dieses Zeitzyklus während der Trainingsphase angegeben wurde.

2. System nach Anspruch 1, wobei der erste Zeitzyklus in mehrere Zeitintervalle unterteilt ist, wobei die Anzahl der Werte der ersten Tabelle (M1) und die Anzahl der Werte der zweiten Tabelle (M2) gleich der Anzahl der Zeitintervalle des Zeitzyklus sind.

3. System nach Anspruch 1 oder 2, wobei die Verarbeitungs- und Steuereinheit (105) so konfiguriert ist, dass sie, falls die in Schritt b) bestimmte Wahrscheinlichkeit unter dem Schwellenwert TH liegt, ein nicht mahlzeitenspezifisches Verfahren zur Regulierung von Blutglukose implementiert (205).

4. System nach Anspruch 3, das ferner eine mit der Verarbeitungs- und Steuereinheit (105) gekoppelte Benutzerschnittstelleneinrichtung (107) aufweist.

5. System nach Anspruch 4, wobei die Verarbeitungs- und Steuereinheit (105) so konfiguriert ist, dass sie, falls in Schritt a) ein Ereignis erkannt wird, vor Schritt b) einen ersten Schritt (203) zur Abfrage des Benutzers mittels der Benutzerschnittstelleneinrichtung (107) durchführt, um ihn zu fragen, ob er innerhalb des Zeitraums T_ANT eine nicht gemeldete Mahlzeit zu sich genommen hat.

6. System nach Anspruch 5, wobei die Verarbeitungs- und Steuereinheit (105) so konfiguriert ist, dass sie:
- falls der Benutzer die erste Abfrage negativ beantwortet, ein nicht mahlzeitenspezifisches Verfahren zur Regulierung von Blutglukose implementiert (205);
- falls der Benutzer die erste Abfrage positiv beantwortet, einen zweiten Schritt (207) zur Abfrage des Benutzers implementiert, und zwar mittels der Benutzerschnittstelleneinrichtung (107), um ihn nach der Zeit und der Größe der nicht gemeldeten Mahlzeit zu fragen, die er während des Zeitraums T_ANT eingenommen hat; und
- falls der Benutzer die erste Abfrage nicht beantwortet, Schritt b) und dann Schritt c) durchführt.

7. System gemäß Anspruch 6, wobei die Verarbeitungs- und Steuereinheit (105) so konfiguriert ist, dass sie:
- falls der Benutzer die zweite Abfrage beantwortet, das Modul zum Mahlzeitenmanagement des Systems zur Regulation aktiviert (209) und die vom Benutzer als Antwort auf die erste Abfrage angegebene Zeit und Größe an das Modul übermittelt; und
- falls der Benutzer die zweite Abfrage nicht beantwortet, auf Grundlage der ersten Tabelle (M1), einen geschätzten Zeitabschnitt zur Mahlzeitaufnahme durch den Benutzer innerhalb des Zeitraums T_ANT bestimmt, und, auf Grundlage der zweiten Tabelle (M2), eine geschätzte Größe der Mahlzeit bestimmt, und dann das Modul zum Mahlzeitenmanagement des Regulierungssystems aktiviert (209) und den geschätzten Zeitabschnitt und die geschätzte Größe der Mahlzeit an das Modul überträgt.

8. System nach einem der Ansprüche 1 bis 7, wobei die Verarbeitungs- und Steuereinheit (105) so konfiguriert ist, dass sie in Schritt c) mittels des Moduls zum Mahlzeitenmanagement einen Insulinbolus bestimmt, der dem Benutzer entsprechend der geschätzten Mahlzeitgröße injiziert werden soll.

9. System gemäß Anspruch 8, wobei die Verarbeitungs- und Steuereinheit (105) so konfiguriert ist, dass sie in Schritt c) den Bolus mit einem Koeffizienten gewichtet, der eine Funktion der in Schritt b) ermittelten Wahrscheinlichkeit ist.

10. System gemäß einem der Ansprüche 1 bis 9, wobei die erste (M1) und die zweite (M2) Tabelle in einem Speicherschaltkreis der Verarbeitungs- und Steuereinheit (105) gespeichert sind.

## Claims

1. Automated blood glucose regulation system, comprising:
- a blood glucose sensor (101);
- an insulin injection device (103); and
- a processing and control unit (105),
wherein the processing and control unit is configured to implement a method of management of undeclared meals, this method comprising the steps of:
a) detecting (201), at a time t0, based on measurements provided by the blood glucose sensor (101) or another indicator, an event likely to correspond to a meal non-declared by a user;
b) when an event is detected at step a), reading (211), in a first table (M1) generated by training from a history of the user's data, a probability for a meal to have been taken by the user within a period T_ANT of predetermined duration preceding time t0;
c) if the probability read at step b) is greater than a threshold TH, reading (213), in the first table (M1), an estimated time slot of meal ingestion by the user within period T_ANT and, reading, in a second table (M2) generated by training from a history of the user's data, an estimated size of the meal, and then activating (209) a meal management module of the regulation system and transmitting to said module the estimated time slot and size of the meal,
wherein:
- the first table (M1) is formed of a series of probability values, each corresponding to a percentage of times that a meal has been declared by the user within a determined time interval of a determined time cycle, during a training phase comprising a plurality of occurrences of said time cycle; and
- the second table (M2) is formed of a series of meal size values, each corresponding to the average meal size declared by the user within each time interval of said time cycle during the training phase.

2. System according to claim 1, wherein the first time cycle is divided into a plurality of time intervals, the number of values of the first table (M1) and the number of values of the second table (M2) being equal to the number of time intervals of said time cycle.

3. System according to claim1 1 or 2, wherein the processing and control unit (105) is configured to, if the probability determined at step b) is lower than threshold TH, implement (205) a blood glucose regulation method non-specific to meals.

4. System according to claim 3, further comprising a user interface device (107) coupled to the processing and control unit (105).

5. System according to claim 4, wherein the processing and control unit (105) is configured to, when an event is detected at step a), implement, before step b), a first step (203) of interrogation of the user, by means of the user interface device (107), to ask them whether they have had an undeclared meal within period T_ANT.

6. System according to claim 5, wherein the processing and control unit (105) is configured to:
- if the user answers negatively to the first interrogation, implement (205) a blood glucose regulation method non-specific to meals;
- if the user answers positively to the first interrogation, implement a second step (207) of interrogation of the user, by means of the user interface device (107), to ask them the time and the size of said undeclared meal taken during period T_ANT; and
- if the user does not answer to the first interrogation, implement step b) and then step c).

7. System according to claim 6, wherein the processing and control unit (105) is configured to:
- if the user answers to the second interrogation, activate (209) the meal management module of the regulation system and transmit to said module the time and the size declared by the user as an answer to the first interrogation; and
- if the user does not answer to the second interrogation, determine (213), based on the first table (M1), an estimated time slot of meal ingestion by the user within period T_ANT and, based on the second table (M2), an estimated size of the meal, and then activate (209) the meal management module of the regulation system and transmit to said module the estimated time slot and size of the meal.

8. System according to any of claims 1 to 7, wherein the processing and control unit (105) is configured to, at step c), determine, by means of the meal management module, an insulin bolus to be injected to the user according to the estimated meal size.

9. System according to claim 8, wherein the processing and control unit (105) is configured to, at step c), weigh said bolus by a coefficient which is a function of the probability determined at step b).

10. System according to any of claims 1 to 9, wherein the first (M1) and second (M2) tables are stored in a memory circuit of the processing and control unit (105).
